# EUROPEAN PATENT SPECIFICATION

(11) **EP 0 345 911 B1**
(45) Date of publication and mention of the grant of the patent: **20.10.2004**
(21) Application number: 89201996.9
(22) Date of filing: 14.06.1984
(51) Int. Cl.: C12N 7/08, A61K 39/215

(54) **Canine coronavirus**
Hundecoronavirus
Coronavirus canin

(30) Priority: 15.06.1983 US 504434; 07.06.1984 US 618638
(43) Date of publication of application: 13.12.1989
(62) Divisional of application: 84902632.3
(73) Proprietor: Wyeth, Madison, New Jersey 07940-0874 (US)
(72) Inventor: Acree, William Marshall, Temple Texas 76501 (US); Edwards, Bobby, Temple Texas 76501 (US); Black, John Wesley, Mitton Tennessee 37118 (US)
(74) Representative: Wileman, David Francis Dr.

(56) References cited:
- VETERINARY MICROBIOLOGY, vol. 7, 1982, pages 427-435, Elsevier Scientific Publishing Co., Amsterdam, NL; D. WOODS: "Studies of enteric coronaviruses in a feline cell line"
- CANINE PRACTICE, vol. 7, no. 4, July/August 1980, pages 22-35; M. APPEL et al.: "Canine viral enteritis"

## Description

The present invention concerns canine coronavirus vaccine, its method of preparation and adjuvants for use therewith.

More particularly the present invention concerns both attenuated and inactivated canine coronavirus vaccines which induce both systemic immunity and local immunity in the intestinal tract.

### BACKGROUND AND FIELD OF THE INVENTION

Canine Coronavirus (CCV) enteritis is a highly contagious disease in dogs with world-wide distribution. CCV gastroenteritis was first observed in February of 1970 in a U.S. Air Force patrol dog training school in Weisbaden, West Germany. In January of 1971, recurrence of the gastroenteritis disease syndrome was observed and the causative agent was identified as a CCV. In 1972 Cartwright and Lucas reported that antibody titers to transmissible gastroenteritis (TGE) virus had significantly increased in dogs after an outbreak of vomiting and diarrhea. It was concluded that TGE or more probably a serologically related virus such as CCV produced disease in these dogs.

Infection results from contact between infected and susceptible dogs. Infected dogs usually show symptoms of disease. However, dogs recovering from disease may shed virus in their feces and prove to be a continued source of infection for susceptible dogs.

In 1978 with the sudden outbreaks of Canine Parvovirus-induced enteritis, a renewed vigor and interest in canine enteric viruses was stimulated. First efforts were directed toward finding the causative agent for this new hemorrhagic enteritis-myocarditis syndrome which was severe and fatal. Upon investigation researchers discovered two distinct viruses, Canine Coronavirus (CCV) and Canine Parvovirus (CPV). After further investigation the CPV proved to be the new agent most often causing the highest morbidity and mortality. The CCV induced a disease somewhat similar but less often fatal. The simultaneous isolation of CPV and CCV has been reported. One survey that was conducted indicated that 17% of the cases of canine enteritis were found to be dual infections with CPV and CCV. Various state diagnostic laboratories confirm these findings.

Differentiation of Parvoviral and Coronaviral Enteritis is difficult. The probable simultaneous infections with both viruses makes differentiation even more difficult. In a serological survey in a kennel situation, 55% of the dogs tested were found to have been exposed to both CCV and CPV. Some kennels tested had up to 87% positive exposure to both agents. The severity of the enteritis attributable to simultaneous infection is unknown. However, one expert researcher in the field proposes that infection by both viruses increases the probability of morbidity in the dog.

As noted above, CCV enteritis is a highly contagious disease which has been observed on a world-wide basis. The incidence of CCV disease in family-owned dogs has been reported to range from 14.8% to 25%. The incidence in kennel-raised dogs ranges upwards to 30%. The incidence of gastroenteritis where both CCV and CPV were isolated is even higher. The disease can occur in dogs of any age. The importance of CCV gastroenteritis has seemingly increased with the outbreak of CPV gastroenteritis.

CCV gastroenteritis is characterized by a number of disease symptoms which have been compiled from the literature as follows: The first signs of disease are lethargy, anorexia and depression. The sudden onset of vomiting occurs in which blood can sometimes be found. Diarrhea can range from moderate to severe and projectile in nature. Diarrhea may persist for up to 10 days. The fecal material has a yellow-orange color with blood and mucus occasionally found therein. Moreover, the fecal material has a marked foul odor. The intestinal tract will contain watery, yellow-green material. Dehydration, weight loss and death have been reported. Protracted or recurring diarrhea may occur 2-3 weeks later. The severity of the CCV disease syndrome is thought to vary according to age, stress, environmental conditions, breed and concurrent infections. CCV has also been associated with respiratory disease symptoms of ocular and nasal discharge.

Both respiratory and enteric symptoms of disease have been seen experimentally by the present inventors. The symptoms observed have included a slight ocular discharge, a slight nasal discharge, diarrhea, weight losses, anorexia, dehydration, elevated temperatures and slight drops in both white blood cell levels and lymphocyte levels. Intestinal samples of infected dogs demonstrated that a significant degree of virus infection occurs following challenge.

CCV is a member of the Coronavirus group of viruses. Some pertinent characteristics of the Coronavirus group include:
a) The virus particle is spherical or elliptical with knoblike projections on the outer surface of 18-20 nanometers (nm).
b) The viruses have very fastidious in vitro growth requirements.
c) The virus particle sizes vary in diameter from 80-200 nm.
d) The virus has a single stranded RNA nucleoprotein.
e) The virus develops in the cytoplasma of the cell.
f) There is a complex antigenic similarity within the group.
g) The virus density in cesium chloride (CsCl) is 1.24 to 1.26 g/mL

Studies show that there do not appear to be distinct antigenic differences among various CCV isolates.

The veterinary community has recognized the need for a CCV vaccine but the literature indicates that little successful work has been done in this area. Experts in the field have recommended an oral-intranasal route of vaccination to insure not only the achievement of systemic immunity but also the obtention of local immunity in the intestinal tract.

The safety of attenuated live virus vaccine must be proven by the developer and licensed by a United States Government Agency. However, misuse of attenuated live virus vaccines or use of attenuated live virus vaccines in a compromised host can lead to field problems. The misuse of product involves, but is not limited to, the combining of several vaccines for simultaneous use when these vaccines have not been studied or recommended for use in this manner. A compromised host includes, but is not limited to, the inoculation of animals undergoing an active infectious process, animals which are in general poor health and animals immunologically incompetent. The immunological incompetence may be present because of immunological deficiency due to inherited genetic traits, because of a concurrent infectious process or treatment with certain chemical compounds, such as dexamethasone.

Some examples of attenuated live virus vaccines which have been licensed for sale but may have caused post vaccinal problems associated with field misuse or use in compromised host are the Modified Live Low Egg Passage Rabies Vaccine, some Modified Live Canine Distemper Vaccines and a Modified Live High Egg Passage Rabies Vaccine. Improved diagnostic techniques have aided in detecting problems associated with attenuated live virus vaccines.

Safety problems in the field due to misuse or due to use in a compromised host can be minimized or eliminated with an inactivated virus vaccine. While inactivated or killed virus vaccines are preferable because of safety they have in the past been of inferior efficacy to attenuated live virus vaccines. While production techniques and adjuvant systems have brought about the development of some highly effective inactivated viral vaccines, the results due to a particular technique or a particular adjuvant system have been unpredictable.

Inactivated virus vaccines have been developed for the most part in the host ranges of farm animals to the feline. The only currently USDA licensed inactivated products for the canine have been used for Canine Parvovirus Enteritis and Rabies. While other inactivated viral products for the canine have been produced they have not met with market acceptance because of inferior effectiveness as compared to corresponding attenuated live virus vaccines.

A problem in the production of an inactivated canine coronavirus vaccine was the general belief that intestinal protection could not be achieved without vaccine viral replication within the target tissue of the intestinal tract. Viral replication cannot be obtained with an inactivated viral vaccine.

It was expected that an oral-intranasal route of vaccination with a live virus vaccine would be required to insure not only the achievement of systemic immunity but also the induction of local immunity in the intestinal tract. Unexpectedly, it has been found that a parenterally administered killed canine coronavirus vaccine induces systemic immunity and also induces local immunity in the intestinal tract.

Appel et al, in Canine Practice, July-August 1980, 4, 22-34, discloses the origin and background of canine viral enteris and particularly describe the effects of CCV and its possible prevention and control. The authors state that at the date of the article a vaccine for protection against CCV was not available.

Other authors, eg Woods, Veterinary Microbiology, 1982, 7, 427-435 and Gill Diss'n Abs. Intl. 43(8), Microbiology, 2452-B (Feb 1983) disclose the propagation but not attenuation of CCV in various cell lines.

It is an object of the present invention to provide attenuated canine coronavirus vaccines which produce both systemic immunity and local immunity in the intestinal tract when administered parenterally.

### SUMMARY OF THE INVENTION

In one aspect the present invention is directed to a new attenuated canine coronavirus, a new method for attenuating canine, coronavirus, a new canine coronavirus vaccine composition, a new method for propagating canine coronavirus, and a new method for evaluating the effectiveness of a canine coronavirus vaccine.

The invention provides attenuated live canine coronavirus which is fully adapted to reproduction and growth in cell culture, which does not produce disease when administered to a dog and which, when administered to a dog by injection, infects intestinal epithelium in the dog to produce localised immunity to canine coronavirus in the intestines.

The invention also provides a method of propagating and harvesting attenuated live canine coronavirus comprising the steps of inoculating a primary or established cell line of feline or canine origin in cell suspension or cell monolayers with an amount of attenuated live canine coronavirus sufficient to achieve a minimum multiplicity of infection (MOI) ratio of at least 1:100 wherein the attenuated live canine coronavirus has been attenuated by passing virulent canine coronavirus in feline cells at least 8 times and wherein the cells are present in an amount sufficient to form a confluent monolayer of the cells within about 48 hours or less of inoculation; culturing in a fluid medium a tight or crowded monolayer of the cells for a period of time less than 96 hours after inoculation sufficient to provide infectivity titers of at least 3 logs of particles of virus per ml as measured by the direct FAID₅₀ method; and harvesting the propagated virus from the cellular material, the fluids or both the cellular material and the fluids.

The original isolation of the virulent CCV was made from a dog which died of gastroenteritis. The isolate was given the designation TN-449. Virus detection methods including electron microscopy indicated CCV as the only virus present in the specimens taken from the dog. This virus isolate was then placed in CRFK cell cultures for propagation and attenuation purposes. A low virus inoculum was placed in CRFK cell cultures which became confluent in 24 hours or less. A virus to cell ratio of about 1 to 3,000 was utilized. The low virus input requires an increased number of virus multiplications which aids in the attenuation process. The virus passaging took about two months. Twelve consecutive cell passages were performed in a heterologous cell culture such as CRFK to achieve attenuation. Dogs were inoculated with various dose levels of passage 12 material. Dose levels of 7.5 log₁₀, 5.5 log₁₀, 3.5 log₁₀ and 1.5 log₁₀ were used parenterally in dogs. A non-inoculated dog was held as a contact control dog. These animals were observed following vaccination. The dogs remained healthy with no symptoms of gastroenteritis being observed. Post inoculation serology verify the virus inoculum had replicated in the dogs. This demonstrated the attenuation of the TN-449 virus. An additional virus passage of the TN-449 was made to establish the Master Seed Virus. The TN-449 culture has been deposited with the American Type Culture Collection in Rockville, Maryland and has been given ATCC Deposit No VR 2068.

The new attenuated (ie modified) live canine coronavirus has three distinguishing characteristics which make it useful as a vaccine virus (1) the attenuated CCV is fully adapted to growth and reproduction in cell culture, especially cells of feline or canine origin, (2) the attenuated CCV does not produce disease when administered to a dog and (3) when administered by injection, eg subcutaneously, parenterally, etc, the virus selectively infects intestinal epithelium. Because of these unique characteristics of the attenuated CCV localized immunity in the intestines is produced when the virus is administered to dogs.

These unique characteristics appear to be due to the method used to attenuate the virus. Attenuated live CCV suitable for use in the present invention is prepared by passing a virulent CCV strain in cells of feline origin at least eight times at a low virus to cell ratio of 1:1,000-10,000, preferably 1:1,500-7,500 wherein the virus particles are measured by the TCID₅₀ method (Tissue Culture Infective Dose).

Passing the virus at a low virus to cell ratio speeds up attenuation and allows the cells to become attenuated with a minimum number of passages. The low virus to cell ratio also acts to genetically select only those cells which are fully adapted to cell culture, thereby producing an attenuated virus strain which is useful in a vaccine. For attenuation purposes, feline cells should be used and canine cells should be avoided. The CCV appears to become attenuated easily in feline cells, however, if canine cells are used a possibility exists that the virus would revert back to the virulent state. Thus, when the attenuated CCV is grown for production purposes, more than one or two passages in canine cells should be avoided. The cells should be passed through the feline cells at least eight times usually 8 to 60 passages and preferably 8 to 25 passages.

The CCV vaccine composition includes an attenuated live virus having a titer of greater than 2.5 logs₁₀ of virus particles/ml (or dose) preferably at least 3.0 logs of virus particles/ml (or dose) and more preferably greater than 3.3 logs of virus particles/ml (or dose) as measured by the FAID₅₀ (Fluorescent Antibody Infectious Dose) method (King et al, Can. Journal of Comparative Medicine & Vet. Science, 29, pp. 85-89 (1965)). Titers as high as 5 to 7 logs FAID₅₀ have been obtained. The CCV vaccine composition includes an attenuated live CCV and also may contain an attenuated live or killed CPV virus. In addition, the vaccine may contain additional attenuated live virus or killed viruses such as Canine Distemper virus, Canine Parainfluenza virus, Canine Adenovirus I, Canine Adenovirus II and Canine Rotavirus. The attenuated live CCV virus produces a complete immunological response to the virus infection. However, the infection produces a fever about 0.5 to 1.0°F (about 0.28 to 0.55°C) lower than the virulent strain of CCV. The vaccine composition contains a sufficient amount of the attenuated live virus to produce an immunological response in a dog and a non-toxic pharmaceutically acceptable sterile carrier or diluent such as a sterile saline solution. Preferably the carrier or diluent is suitable for parenteral injection, however, the carrier or diluent may also be of a type which is suitable for use as a nasal spray.

The method for propagating the CCV in mammalian cells includes the steps of inoculating mammalian tissue culture cells with CCV, cultivating the cells into a one hundred percent (100%) confluent tight monolayer before said inoculating step or within 24 hours of said inoculating step, harvesting the cells between 24 and 96 hours after inoculation and collecting the propagated virus from the harvested cells.

The mammalian tissue culture cells are inoculated with CCV at a CCV virus (measured by the FAID₅₀ method) to cell ratio of 1:500 to 1:1, preferably 1:100 to 1:5, more preferably 1:75 to 1:10. The mammalian tissue culture cells include but are not limited to Crandall Feline Kidney Cells (CRFK), Wood's Feline Cell Line (FC), a Dog Kidney Cell Line (DK), Madin Darby Canine Kidney (MDCK) and the canine A72 cell line. The CCV is added to a suspension of the cells or is applied to a monolayer of the cells. The amount of cells and virus should be such that a confluent tight monolayer of the cells will form within 12-48, preferably 24-36, hours after inoculation. Optimally, at the time of inoculation the cells should be present in the growth vessel in an amount sufficient to form a monolayer of cells of at least 100,000 to 1,000,000 cells per square centimeter (cm²) within about 12-48 hours after inoculation, preferably within 24 hours after inoculation. However, a lesser number of cells may be used. Preferably the cells are present in an amount sufficient to form between 150,000 to 500,000 cells/cm² within 12-48 hours, preferably within 24 hours. The virus is adsorbed on the cells for at least 60 minutes but usually less than 300 minutes, preferably between 90 and 240 minutes at 28 to 40°C, preferably 35 to 38°C.

Harvestable virus titers of at least about 1,000 and usually at least about 2,000 as measured by the FAID₅₀ method can be obtained within 18 hours after inoculation, however, in some circumstances it may take up to 120 hours or longer to obtain maximum virus titers. Maximum virus titers are usually obtained within about 24 to 96 hours after inoculation. The cell monolayer is removed either by freeze-thawing or by enzymatic action to increase the viral content of the harvested fluids. The harvested fluids are then combined with a virus stabilizer such as sucrose phosphate glutamate (SPG), sucrose phosphate glutamate albumin (SPGA) or NZ amine-gelatin for final product filling or frozen in bulk for later thawing and bulking with virus stabilizer. Alternatively, the harvested fluids may be inactivated.

The vaccine composition comprises a sufficient amount of the canine corona-virus antigen to produce an immunological response in a dog and a non-toxic pharmaceutically acceptable adjuvant or adjuvant combination.

The induction of humoral-systemic protection as measured by serum neutralizing antibody levels as well as the induction of local immunity in the intestinal tract is an important feature of this invention. The oral-intranasal routes of administration should elicit both types of protection. However, the general marketplace acceptance of this route of administration is not good and, therefore, the use and subsequent protection afforded by a CVV vaccine would not be realized. An attenuated live virus vaccine which is parenterally administered will provide humoral and localized immunity. The parenteral vaccination with an inactivated CCV vaccine will also induce both humoral immunity and localized immunity. The term parenteral vaccination is intended to include subcutaneous and intramuscular inoculation routes.

The attenuated vaccine is usually parenterally administered in a dose of at least 2.5 logs of virus particles per dose, preferably at least 1,000 virus particles (3 logs of virus particles) per dose, more preferably at least 3.3 logs of virus particles per dose.

The vaccine may be administered in either a one or two dose regimen. The second dose should be no more than six weeks after the first. Preferably the second dose is administered two to three weeks after the first dose.

Individual or combinations of various adjuvants may be utilized to inhance the immune response. Adjuvants such as, but not limited to, aluminum phosphate, ethylene maleic anhydride, Neocryl A640, and aluminum hydroxide may be utilized. Neocryl is a trade name for a latex emulsion of a copolymer of styrene and a mixture of acrylic acid and methacrylic acid. Neocryl A640 is an uncoalesced aqueous acrylic copolymer with styrene, having pH 7.5, viscosity 100cps (Brookfield 25° c), weight per gallon is 8.6 pounds as supplied containing 40 percent solids by weight and 38 percent solids by volume. The numeral A640 denotes a grade thereof. Other useful Neocryl grades are 520, 625, and 966. The term "CSMA" will be used hereinafter to refer to a copolymer of styrene with a mixture of acrylic acid and methacrylic acid.

Ethylene maleic anhydride (EMA) prepared at a 5 percent weight/per voL concentration in water added to the inactivated virus fluids at 0.01 percent to 6 percent volume to volume concentration. The pH of the resulting fluids is adjusted to to 7.7 by addition of 1 normal sodium hydroxide.

CSMA prepared in a 50 percent volume per volume suspension in water is added to the inactivated CCV fluids from 0.1 percent to 10 percent volume. Usually there is no need for a pH adjustment as the CSMA is of a neutral pH.

Combining of two or more adjuvants may be accomplished as in the following, but are not limited to this combination. Ethylene maleic anhydride as described is added at 0.01 percent to 6 percent volume to volume to the inactivated virus fluids. The mixture is adjusted to a 7.1 to 7.7 pH by addition of 1 normal sodium hydroxide. The addition of 0.01 percent to 10 percent of the CSMA, as described, is accomplished with no further pH adjustments being required.

A method utilized to evaluate the effectiveness of a CCV vaccine includes the steps of vaccinating a dog with a modified live CCV vaccine, bleeding the vaccinated dog after the dog has developed an immunological response to the vaccine, separating the serum from the blood, challenging the vaccinated test dog and a non-vaccinated dog with an infectious amount of a virulent CCV by an intranasal-oral route, and examining intestinal tract samples of the vaccinated dogs and the control dogs to determine the degree of replication of the challenge virus. The United States Department of Agriculture (USDA) has standard testing requirements for determining the host animal effectiveness of some veterinary products. No standard requirement, nor proposed testing requirements, are in existence for CCV vaccines. Furthermore, no adaptations of standard or proposed testing procedure were possible to validly evaluate the effectiveness of the product in host animals. Therefore, a new test method for evaluating the effectiveness of the CCV vaccines has been developed. This procedure involves the direct measure of protection afforded in the intestinal tract of vaccinated animals against infection of the intestinal tract with virulent Canine Corona challenge virus. In general, vaccinated dogs are bled at least 7 and preferably 7 to 21 days following vaccination to determine the humoral protection afforded by measuring the quantity of serum-neutralizing antibody present in the serum. These dogs, along with non-vaccinated control dogs, are then challenged with virulent CCV by the intranasal-oral route. The intestinal tract samples of both vaccinated and control dogs are examined to determine the degree of challenge virus replication which has taken place. Sampling can be performed between 2 and 14, preferably between 3 and 10 days after challenge. Direct impression smears, intestinal scrapings, or processed intestinal scrapings can be used for evaluation purposes.

The detection of virus in the intestinal sample includes but is not limited to both direct and indirect fluorescent antibody staining. The measure of vaccine effectiveness for providing local immunity is measured by the difference in the degree of infection between vaccinated and control dogs. A reduction of 90% is not unusual.

### DETAILED DESCRIPTION OF THE INVENTION

The present invention is based upon several discoveries. One discovery is the method of propagation used for the Canine Coronavirus which yields sufficiently high titers for blending purposes as required in vaccine preparation. The propagation of Canine Coronavirus to high virus infectivity levels can be difficult so that this aspect of the invention is extremely important. The methods of production developed yield virus infectivity titers of at least 3 logs of virus per ml. as measured by the direct FAID₅₀ method.

A further point to be noted in connection with this aspect of the invention is that the CCV titers achieved by the method described herein are sufficient to blend with virus stabilizer components but also of sufficient quantity to allow for the combining of CCV with other virus agents such as but not limited to Canine Parvovirus, Canine Distemper virus, Canine Adenovirus I, Canine Adenovirus II, Canine Parinfluenza, Canine Rotavirus, Leptospira canicola and Leptospira icterohemorrhagiae.

Some major factors in achieving high titer coronavirus are the virus cell ratio adsorption time of virus and cells, cell contact inhibition obtained, and the harvest time of virus fluids following inoculation.

An attenuated live CCV is used in conjunction with either primary cell cultures or an established cell line of either canine or feline origin. Incubation temperatures of 28-40°C are employed with 35-38°C being preferred. An MEM-Earles tissue culture media supplemented with an animal serum such as bovine is used. A minimum concentration of 5-10% is used for cell culture growth and a concentration of not less than 1% is used in the maintenance medium. A minimum concentration of 0.5 ml of an enzyme such as crude or purified trypsin per liter of tissue culture medium is used. Cell cultures are planted in either stationary or roller culture bottles.

In the case of cell monolayers, a cell population of not less than 150,000 cells per square centimeter is used. The monolayer appears tight and confluent, thereby giving the contact cell inhibition needed. The cell culture growth media is removed and seed virus is added. Frozen CCV seed is thawed in cold water and placed in at least a sufficient volume (such as 50 ml) on the cell monolayer for an adsorption period of at least 90 minutes at temperatures between 35° and 38°C. A minimum virus to cell ratio of 1 virus particle to every 100 cells is essential.

In the case of a cell suspension, sufficient cells are used per a specific surface area to ensure that a crowded, confluent monolayer is obtained in 24 hours or less. A cell culture passage of 1 vessel of uninoculated cells to 1 or 2 vessels for infected cells may be used.

The time of harvest is critical in obtaining sufficient titers of CCV for vaccine blending. An infectivity titer of at least 3.3 logs of virus per ml is needed. The harvest times required to obtain virus titers of this magnitude range from 24 hours after infecting the cell population to 96 hours after cell population infection. Virus infectivity titers peak at 48 hours and decline thereafter. However, sufficient infective virus will remain up to about 96 hours. Virus infectivity titers can be increased by at least two fold by removing the remaining cell monolayer by enzymatic action or by freeze-thawing and adding the cell suspension to the previously harvested virus fluids.

Another major discovery of this invention is that the vaccine virus will provide humoral, systemic protection as measured by serum neutralizing antibody levels and localized intestinal protection as measured by vaccination-challenge studies when the vaccine is administered parenterally. Unexpectedly, it was found that induction of localized immunity has been associated with routes of vaccination other than parenteral and the possibility of providing local immunity to the intestinal tract by parenteral vaccination with CCV was deemed unlikely. The systemic immunity is likely due primarily to the presence of gamma--immunoglobulin (IgG) and the local immunity in the intestinal tract is likely due at least partially to the presence of alpha-immunoglobulin (IgA) as well as IgG.

Experimentation with various dosage levels of CCV vaccine given by the parenteral route of vaccination demonstrated vaccine viral replication in the intestinal tract as early as five days after administration and a serological response as early as 7 days after administration. Vaccinated dogs, when challenged, indicated a reduction of disease symptoms and up to a 95% reduction in intestinal infection as compared to unvaccinated controls. The data thus supports the uniqueness of this portion of the discovery that parenterally administered CCV vaccine provides systemic, humoral protection as well as local immunity in the intestinal tract.

Another discovery of this invention comprises the means by which the vaccine is evaluated in canines. The vaccine is designed to prevent systemic and localized gastroenteritis infections induced by the CCV. Circulating or humoral antibody evaluations were conducted on blood samples taken 7 to 21 days after parenteral vaccination with a one milliliter dose of vaccine per dog. At 21 days after vaccination, vaccinated dogs and non-vaccinated dogs were intranasally-orally challenged with virulent Canine Coronavirus. The onset of disease is rapid and sudden. Disease symptoms are seen as early as 24 hours after challenge in susceptible dogs. The duration of symptoms is usually 24-96 hours, but can extend to 10 days. In evaluating the effectiveness of the vaccine, the animals are euthanized at 5, 6, or 7 days after challenge. The intestinal tract from the pyloric valve to the large intestines is removed. The intestines are processed and examined by impression smear scrapings or exfoliated intestinal epithelium is used.

The impression smears are made by scraping the epithelial lining systematically at several locations throughout the intestine. The scrapings are put on slides and processed for direct fluorescent antibody staining to detect CCV infected cells. The same epithelial scraping may be exfoliated by placing the scraping in phosphate buffered saline. The epithelium saline mixture is agitated to suspend the individual cells in the saline. Then, samples of the suspensions are placed on slides to be processed for direct fluorescent antibody (FA) staining for CCV.

The examination of the slides reveals the extent of specific fluorescence as a measure of virus infection. The degree of fluorescence based on the number of infected cells per field in conjunction with the number of samples examined per dog yield a numerical value of infection which can be assigned to each individual animal. Thus, evaluation as to degree or extent of CCV infection in the intestinal tract is obtained. Comparison of the values from vaccinated and non-vaccinated dogs yields a means of evaluating the effectiveness of the vaccine in preventing intestinal infection.

In order more clearly to disclose the nature of the present invention, specific examples of the practice of the invention are hereinafter given. It should be understood, however, that this is done solely by way of example and is intended neither to delineate the scope of the invention nor limit the ambit of the appended claims. In the examples, all temperatures are stated in degrees centigrade, logs means logs to the base 10, and the following abbreviations are used: g." for grams, "mcg." for micrograms, "ml." for milliliters, "min." for minutes, and "hr." for hours.

### EXAMPLE 1

### This example illustrates vaccine production.

The production strain was orginally isolated from a dog which had died of CCV enteritis. The virus was serially propagated in Crandall Feline Kidney Tissue (CRFK) culture. Upon receipt the virus was designated CCV-MSV and passaged once to Master Seed CCV-MSV(X). The CCV-MSV (Canine Corona Virus-Master Seed Virus) culture has been deposited with the American Type Culture Collection in Rockville, Maryland and given ATCC Deposit No. VR 2068. In vaccine production the cell cultures were grown in dynamic cultures. In some preliminary runs, static cultures were used. Cell cultures were grown in minimal essential media (MEM) supplemented with vitamins, non-essential amino acids, sodium pyruvate, sodium bicarbonate and L-glutamine. The amount of 30 mcg/ml. of gentamicin was added as a preservative. A 5-10 percent by weight concentration of bovine serum was added for cell growth. The serum concentration was reduced to not greater than 1 percent for a maintenance medium. Trypsin was added at a concentration of 0.5 ml./liter of medium to promote virus infectivity.

Confluent cultures of CRFK cells were trypsinized and planted into roller cultures so that a density of 150,000 cells per cm² was obtained after 24 hours. Cultures were grown at 28 to 40°C, preferably 35-38°C. The growth media was removed.

The production seed virus was thawed in cool running water. Sufficient virus was added to achieve a minimum multiplicity of infection (MOI) ratio of 1:100. A 2000 cm² bottle contained 300-500 million cells. At a 1:50 MOI 300 x 10⁶ divided by 50 yielded a minimal infective virus inoculum of 6 x 10⁶ or 10^{6.8}FAID₅₀ per roller. Virus seeds and product harvests yielded 10^{5.5} to 10^{7.0} FAID₅₀ per ml. Thus 20 ml. of undiluted to 1 ml. of undiluted seed was usually used per bottle. The seed was brought up to a volume of 50 ml. per roller bottle with culture medium. The virus was allowed to adsorb on monolayers or in suspension for 2 hours at 35 to 38°C.

A specific example used a seed titered at 10^{5.7}FAID₅₀ per ml. with 15 ml. per roller used as the inoculum. Thirty two roller bottles were inoculated as described. The bottles were refed with 2 liters of MEM at 1 percent FCS and 0.5 ml of trypsin. The fluids were harvested along with the cellular material 48 hours after infection, dispensed and frozen at -40°C or lower.

The bottles yielded 66.5 liters of virus fluids. The titer of the fluids was 10^{5.0}FAID₅₀ₛ per ml. The material was later thawed and blended with media and sucrose phosphae glutamate albumin (SPGA) stabilizer and lyophilized.

### EXAMPLE 2

### This example illustrates intestinal propagation through parenteral inoculation.

The purpose of the study was to determine whether the CCV vaccine would locate the replicate in the intestinal tract when administered intranasally or intramuscularly to healthy susceptbile dogs.

Four dogs were divided into two groups, isolated, and bled prior to vacination. Dogs Nos. 63 and 64 were vaccinated intramuscularly with a 10 ml. dose (10^{6.3} FAID₅₀/dose) of CCV (x + 1) seed (seed prepared by one additional passage from the master seed). The two other dogs Nos. 62 and 65 were vaccinated intranasally with a 2 ml dose (10^{5.6} FAID₅₀/dose) of the same CCV seed. On day 5 after vaccination the dogs were bled, swabbed for fecal samples for virus isolation in CRFK cell culture, and euthanized Tissue scrapings from the trachea, duodenum, jejunum and cecum were placed on slides. These impression smears were then subjected to observation for virus replication by the direct fluorescent antibody technique.

### Fecal Virus Isolation

No Coronavirus could be isolated from the feces.

### Direct FA Staining of Impression Slides

The results of fluorescent antibody staining of tissue impression slides are shown in Table 2-1.

**TABLE 2-1**

| CCV Direct FA of Impression Slides | | | | | | | | |
|---|---|---|---|---|---|---|---|---|
| Dog No. | Virus Input (FAID)₅₀/dose | Vaccination Route | CCV+ slides/No. of slides taken | | | | CCV+Slides/Total Slides | CCV Positive (%) |
| | | | Trachea | Duodenum | Jejunum | Cecum | | |
| 63 | 10^{6.3} | IM | NA | 10/12 | 4/7 | 3/5 | 17/24 | 71 |
| 64 | 10^{6.3} | IM | NA | 3/6 | 4/5 | 4/5 | 11/16 | 69 |
| 62 | 10^{5.7} | IN | 3/4 | 2/2 | 2/2 | 2/2 | 6/6 | 100 |
| 65 | 10^{5.7} | IN | 2/2 | 2/2 | 4/4 | 5/5 | 11/11 | 100 |

One hundred percent of the intestinal impression slides taken from the intranasally inoculated dogs were positive for virus replication, while seventy percent of the intestinal impresson slides were positve for virus replication from the dogs inoculated intramuscularly.

Virus replication occured in all three sections of the intestinal tract. Virus replication was also observed in the trachea of the dogs vaccinated intranasally.

The degree of virus involvement in the intestine of intranasally inoculated animals and the fact that the trachea also exhibited virus replication may indicate this route of inoculation as the natural portal of entry for infection.

### Conclusion

Virus replication in the intestinal tract can be achieved 5 days afer administering high dose levels of virus either intramuscularly or intranasally.

### EXAMPLE 3

### This example illustrates low virus inoculum parenterally administered for intestinal replication.

The purpose of this study was to determine whether the CCV vaccine when administered intramuscularly in a low dose level would locate and replicate in the intestinal tract.

Two dogs Nos. 60 and 68 were vaccinated intramuscularly with 1 ml. of the CCV-MSV (x + 3) seed diluted to 10^{3.0}FAID₅₀/ml. On day 5 after vaccination the dogs were bled and euthanized. Impression smears from villi scrapings were made from the duodenum, jejunum and cecum for direct FA observation to detect virus replication. These smears were processed and stained with CCV specifc FITC (fluoroisothiocyanate) bound conjugate and examined via FA.

### Direct CCV FA from Villi Scrapings

Recovery of positive CCV from the villi scraping slides are shown in Table 3-l.

**TABLE 3-1**

| CCV Direct FA of Villi | | | | | | | |
|---|---|---|---|---|---|---|---|
| Dog No. | Virus Input | Vaccination Route | CCV+ slides/No. of slides taken | | | CCV+Slides/Total Slides | CCV Positive (%) |
| | | | Duodenum | Jejunum | Cecum | | |
| 60 | 10^{3.0} | IM | 6/7 | 5/7 | 6/6 | 17/20 | 85 |
| 68 | 10^{3.0} | IM | 4/6 | 5/6 | 4/6 | 13/18 | 72 |
| | | | | | | | Avg. 96=79 |

CCV replication was found in all areas of the intestinal tract of both dogs 5 days following the intramuscular administration of 3 logs of virus. An average of 79% of the samples observed were positve for virus replication. This percentage compares favorably with the 70% positive sample obtained with dogs administered with 5.6 logs of virus in another study.

CCV replication can be demonstrated in the intestinal tract of susceptible dogs vaccinated parenterally with 10^{3.0}FAID₅₀/dose of CCV vaccine 5 days after vaccination.

### EXAMPLE 4

### Preliminary Vaccine and Challenge Evaluation

The purpose of this study was to evaluate a Canine Corona Challenge Virus and to determine if the vaccine virus will prevent any symptoms induced by the challenge virus and reduce or prevent intestinal infection by the challenge virus.

Two Canine Corona virus-susceptible dogs, Nos. 61 and 66, were bled and vaccinated intramuscularly with 3 logs of CCV MSV (x + 3) seed. These animals were held in isolation until the time of challenge.

At 21 days after vaccination the two CCV vaccinates along with the four CCV susceptible dogs Nos. 75, 79, 74 and 76 were bled, anesthetized and challenged with 5 ml./dog of CCV challenge virus. Each dog received 2 ml. orally and 3 ml. intranasally. Each dog received 10^{5.7} FAID₅₀ of challenge virus.

Dogs Nos. 74 and 76 were monitored for 7 days after challenge for temperature response, WBC (white blood cell) count, lymphocyte count response and other symptoms. At 5 days after challenge the two CCV vaccinates Nos. 61 and 66 along with the two challenge control dogs Nos. 75 and 79 were euthanized. Intestinal scraping impression smears were made of the duodenum, jejunum and cecum of each dog. Fluorescent antibody staining was performed on these smears to determine the extent of viral replication in the intestinal tract.

**TABLE 4-1**

| CCV SN Testing | | | |
|---|---|---|---|
| Dog No. | Group | CCV Serum Neutralizing (SN) Antibody Titer | |
| | | Pre-vaccination | Pre-Challenge |
| 61 | V* | < 1:2 | 1:1122 |
| 66 | V | < 1:2 | 1:1122 |
| 75 | C** | | < 1:2 |
| 79 | C | | < 1:2 |
| 74 | C | | < 1:2 |
| 76 | C | | < 1:2 |

| | | | |
|---|---|---|---|
| * Vaccinate | | | |
| ** Challenge Control | | | |

Both vaccinates were seronegative prior to vaccination and serocoverted to a 1:1122 level 21 days after vaccination.

An average of 95.6 percent of all intestinal samples observed in the controls demonstrated a 4(+) virus replication level. None of the vaccinates demonstrated a 4(+) level of virus infection in the intestinal samples. Therefore a 100% reduction in the 4(+) level of infection was achieved.

A total of 17.4 percent of the vaccinate samples were positive with a 1(+) level of infection whereas a total of 100% of the challenge control samples were positive (95.6% at 4+, 4.4% at 1+). An overall percent reduction in intestinal infection of 82.6% was achieved in the vaccinates.

Further analysis of this data can be performed which gives significance to the 4+ level of infection in comparison with a 1+ level of infection. This analysis can be found in Table 4-2.

**TABLE 4-2**

| Dog No. | Degree of Infection | Number of Intestinal samples (+) per group | Number of samples multiplied by the degree of infection | Total Number per group or Infective Index |
|---|---|---|---|---|
| Controls | | | | |
| 75 | 4+ | 22 | 88 | 178 |
| | 1+ | 1 | 1 | |
| | 0 | 0 | 0 | |
| 79 | 4+ | 22 | 88 | |
| | 1+ | 1 | 1 | |
| | 0 | 0 | 0 | |

| Vaccinates | | | | |
|---|---|---|---|---|
| 61 | 4+ | 0 | 0 | 8 |
| | 1+ | 7 | 7 | |
| | 0 | 16 | 0 | |
| 66 | 4+ | 0 | 0 | |
| | 1+ | 1 | 1 | |
| | 0 | 22 | 0 | |

### CCV Challenge Evaluation Results and Discussion

### 1. Temperature Response

| Post CCV Challenge Temperature Response (100°F) | | | | | | | | |
|---|---|---|---|---|---|---|---|---|
| | | Day Post Challenge | | | | | | |
| Dog No. | Group | 0 | 1 | 3 | 4 | 5 | 6 | 7 |
| 74 | Monitored Challenge Control | 2.2 | 2.5 | 3.6* | 4.4 | 2.8 | 2.0 | 2.0 |
| 76 | " | 2.0 | 2.2 | 4.0* | 4.2 | 3.2 | 2.6 | 2.0 |
| AVG. | | 2.1 | 2.4 | 3.8 | 4.3 | 3.0 | 2.3 | 2.0 |
| 75 | Euthanized Challenge Control | Not taken until severe temp. noted in Nos. 74&76 | | 3.0 | 4.0* | 3.5* | Euthanized for Gut FA | |
| 79 | " | " | | 4.6* | 4.0* | 3.4 | " | |
| AVG: | | | | 3.8 | 4.2 | 3.2 | | |
| 61 | CCV | " | | 2.2 | 2.0 | 1.6 | " | |
| 66 | " | " | | 2.6 | 3.0 | 2.6 | " | |
| AVG. | | | | 2.4 | 2.5 | 2.0 | | |

| | | | | | | | | |
|---|---|---|---|---|---|---|---|---|
| *Critically high fever | | | | | | | | |

The CCV challenge produced a critically high fever (103.5° F) in the CCV susceptible challenge control dogs for day 3, 4, 5 respectively. All of the four challenge control dogs expressed temperatures of 103.5F for two consecutive days. Dogs 74 and 76 averaged temperature of 103.8F and 104.3F on day 3 after challenge and 104° F on day 4 after challenge. Dog 75 had a delayed temperature response of 104° F and 103.5° F on days 4 and 5 after challenge.

The CCV vaccinated dogs displayed no temperature over 103° F for the 3 days they were monitored during the critical fever period. The CCV challenge produced a significant febrile response in CCV susceptible dogs and the CCV vaccine prevented this temperature increase.

### 2. WBC Response

| WBC Count Post CCV Challenge (X100) | | | | | | | |
|---|---|---|---|---|---|---|---|
| | Days Post Challenge | | | | | | |
| Dog No. | 0 | 1 | 3 | 4 | 5 | 6 | 7 |
| 74 | 195 | 252 | 178* | 125* | 125* | 134 | 169 |
| 76 | 247 | 177 | 130* | 85* | 105* | 155 | 150 |

| | | | | | | | |
|---|---|---|---|---|---|---|---|
| *critical days | | | | | | | |

| Percentage WBC Drop Post CCV Challenge | | | | | | | |
|---|---|---|---|---|---|---|---|
| Dog No. | 1 | 2 | 3 | 4 | 5 | 6 | 7 |
| 74 | 0 | - | 8.7 | 35.9 | 35.9 | 31.3 | 13.3 |
| 76 | 28 | - | 47 | 65.6 | 57.5 | 37.2 | 39.3 |

The critical period for a WBC drop following CCV challenge was on days 3, 4 and 5. This data indicates WBC monitoring may be a critical parameter for the Canine Coronavirus disease syndrome.

### 3. Lymphocyte Count

| Lymphocyte Count Post CCV Challenge (X100) | | | | | | | |
|---|---|---|---|---|---|---|---|
| | Days Post Challenge | | | | | | |
| Dog. No. | 0 | 1 | 3 | 4 | 5 | 6 | 7 |
| 74 | 82 | 103 | 43* | 39* | 31* | 56 | 61 |
| 76 | 131 | 58* | 46* | 29* | 29* | 68 | 65 |

| | | | | | | | |
|---|---|---|---|---|---|---|---|
| *critical days | | | | | | | |

A lymphopenia developed in the CCV challenge control dogs beginning on day 1 with dog 76 and day 3 with dog 74. The lymphopenia continued through day 5 after challenge. Further analysis of the percent lymphocyte drop is listed below:

| Percentage Lymphocyte Drop Post CCV Challenge | | | | | | |
|---|---|---|---|---|---|---|
| | Days Post Challenge | | | | | |
| Dog. No. | 1 | 3 | 4 | 5 | 6 | 7 |
| 74 | - | 48 | 52* | 62* | 32 | 26 |
| 76 | 36* | 65* | 78* | 78* | 48 | 50 |

| | | | | | | |
|---|---|---|---|---|---|---|
| *critical days | | | | | | |

Lymphopenia with over a 50% reduction in lymphocytes occurred in 6 of 12 observations of the challenge control dogs with the most severe occurrences on days 4 and 5 after challenge. Dog 74 had greater than 50% lymphopenia on days 4 and 5 after challenge. Dog 76 displayed a lymphopenia over 50% on days 3, 4, 5 and 7 after challenge and had the largest drop of 78% on days 4 and 5 after challenge.

### 4. Symptoms

| Symptoms Post CCV Challenge | | | | | | | |
|---|---|---|---|---|---|---|---|
| | Days Post Challenge | | | | | | |
| Dog No. | 0 | 1 | 3 | 4 | 5 | 6 | 7 |
| 74 | None | None | Nasal Discharge Anorexia Dehydration Feces Dry | Slight Nasal Discharge Slight Ocular Discharge Anorexia Dehydration Feces Wet | Soft Stool | Slight Nasal Discharge | Slight Nasal and Ocular Discharge |
| | | | | | | | |
| 76 | None | None | Same as Above | Same as Above | Slight Nasal Discharge Feces Soft | Same as Above | Same as Above |

Symptoms of CCV infection began on day 3 after challenge and ran concurrent with the febrile response and lymphopenia. Symptoms of nasal discharge, anorexia, dehydration, and ocular discharge continue through day 7 after challenge, the last day of monitoring. The CCV challenge produced significant CCV symptoms in CCV susceptible dogs.

### Evaluation of Virus Replication in the Intestinal Tract of Vaccinates and Controls 5 Days After Challenge

A total of 8 locations were selected in the trachea for impression slide preparations and 23 locations were selected along the intestinal tract for evaluation. Intestinal scrapings were placed on slides which were acetone fixed and stained with specific Canine Coronavirus FA conjugate. The degree of replication was evaluated as to a 4+ infection, 1+ infection or no virus present. The results of these evaluations are shown in Tables 4-3 and 4-4.

**TABLE 4-3**

| Sample Evaluation for Canine Coronavirus Replication | | | | | | | |
|---|---|---|---|---|---|---|---|
| Group | Dog No. | Degree of FA+ | No. of Slides | | | | |
| | | | Trachea | Duodenum | Jejenum | Cecum | TOTAL |
| Challenge control | 75 | 4+ | 0 | 6 | 8 | 8 | 22 |
| | | 1+ | 0 | 1 | 0 | 0 | 1 |
| | | No CCV | 8 | 0 | 0 | 0 | 8 |
| Challenge control | 79 | 4+ | 0 | 6 | 8 | 8 | 22 |
| | | 1+ | 0 | 1 | 0 | 0 | 1 |
| | | No CCV | 8 | 0 | 0 | 0 | 8 |
| Vaccinate 61 | 61 | 4+ | 0 | 0 | 0 | 0 | 0 |
| | | 1+ | 0 | 2 | 4 | 1 | 7 |
| | | No CCV | 8 | 5 | 4 | 7 | 24 |
| Vaccinate 66 | 66 | 4+ | 0 | 0 | 0 | 0 | 0 |
| | | 1+ | 0 | 0 | 0 | 1 | 1 |
| | | No CCV | 8 | 7 | 8 | 7 | 30 |

No Trachea virus replication was noted in any of the dogs. Further evaluation of the percent of positive samples will exclude the trachea samples.

### EXAMPLE 4-4

| Dogs | Percent of samples FA(+) per degree of infection in the intestinal tract | | | Average Total % per test group | |
|---|---|---|---|---|---|
| | 4+ | 1+ | 0 | (+) | (-) |
| 75 | 95.6 | 4.4 | 0 | | |
| 79 | 95.6 | 4.4 | 0 | | |
| Control Average | 95.6 | 4.4 | 0 | 100 | 0 |
| 61 | 0 | 30.4 | 69.6 | | |
| 66 | 0 | 4.4 | 95.6% | | |
| Vaccinate Average | 0 | 17.4 | 82.4 | 17.4 | 82.4 |

An infective index level of 178 for the controls versus 8 from the vaccinates indicates a reduction in infection of 95.5%.

The study thus establishes that the CCV challenge produced severe measurable clinical symtoms of CCV disease and the CCV challenge can be observed in the gut. The CCV vaccine lowers the degree of infection in the vaccinated dog's gut and eliminates the clinical symptoms and fever after CCV challenge when compared with CCV susceptible challenge control dogs.

### EXAMPLE 5

### This example illustrates Vaccine Efficacy.

Pups were proven seronegative to CCV by a serum neutralization test via F.A. Twenty-two of the animals were vaccinated with a minimal dose of vaccine. One half of the group was given the vaccine subcutaneously and the other half of the group was administered vaccine intramuscularly. The vaccinated dogs then were housed individually but kept in the same area along with three more pups, which served as environmental controls. The animals were monitored and held for three weeks. No untoward effects from the vaccine were displayed and there were no signs of environmental exposure to virus.

Each vaccinate was housed so as to prevent direct dog to dog contact. This procedure was employed to prevent a vaccinate, which may be shedding, from inadvertently exposing another vaccinate which might not be immunized at the time of vaccination. The environmental controls were housed in the same area but kept physically separate from the vaccinates. These animals were bled periodically throughout the observation time and euthanized at day 2L Intestinal scraping impression slides were made and evaluated by direct fluorescent antibody staining. Serological evaluation of the serum samples drawn and evaluation of the intestinal scraping impression slides determined whether environmental exposure to a Canine Coronavirus had ocurred.

At 21 days after vaccination the vaccinates and controls were anesthetized, bled and challenged intranasally-orally with virulent challenge. The challenge virus was titered to determine the virus input. The vaccinates, controls and challenge sentinel controls were housed in the same building. The vaccinates and controls were monitored daily for temperature elevation, lymphopenia, leukopenia and other symptoms of Corona-induced disease.

A random selection of vaccinates and controls was performed for euthanizing and evaluation of the degree of challenge virus replication in the intestinal tract on days 5, 6 and 7 following challenge. The challenge sentinel controls were euthanized and slides of intestinal tract scraping examined on days 6 and 7.

All test animals were housed in rooms where the temperature ranged from 50-70° F following challenge.

### Results

### A. Virus Input (See Table 2-1)

Ten replicate titrations were performed on the virus used to vaccinate the initial set of vaccinates. A geometric mean titer of 2.9 logs of virus was used.

### B. Comparison of Antibody Response and Overall Geometric Means

A geometric mean titer of 1:138,491 was obtained by the subcutaneous route of vaccination: The intramuscular route of inoculation elicited a geometric mean serological response of 1:113,761. No significant difference was evident between the routes of administration and the serological response obtained.

### C. Vaccine Sentinel Controls (See Table 5-1 ad 5-2)

The three dogs used as sentinel controls were shown seronegative at day 0 and at the conclusion of the study.

These three dogs were sacrificed and their intestinal tracts examined for infection by the fluorescent antibody staining of intestinal scraping impression slides. The eighteen samples examined for each animal were negative for Canine Coronavirus.

It was concluded no "wild type" Canine Coronavirus contaminated the facility to invalidate the study.

### D. Titration of the Challenge Virus

An isolate of Canine Coronavirus was obtained for intranasal oral administration to each vaccinate and challenge control dog. A geometric mean of 5.47 logs per ml. was obtained from 7 titrations. Each animal received 2 ml. of virus intranasally and 3 ml. orally. Therefore, each animal was challenged with 6.2 logs of virus.

### E. Observations and Evaluations Following Challenge

### 1. Temperature Response

One hundred thirty-one observations were made for the subcutaneously and intramuscularly vaccinated groups. No temperature response greater than 103°F was observed.

Fifty-seven temperature observations were made on the challenge control dogs. A total of 6 observations or 9.5% of the observations were over 103° F. These temperatures were 103.2, 103.2, 103.4, 103.6, 104.0 and 105.0, respectively.

The small degree of temperature response noted in the challenge control dogs was not present in the vaccinates.

### 2. White Blood Cell (WBC) Response

No significant drop in WBC was observed in either vaccinate or challenge control dogs.

### 3. Lymphocyte Response

There appeared to be a significant drop in lymphocytes for the challenge control dogs. Seven of ten challenge control dogs or 70% had lymphocyte drops of greater than or equal to 60%. Only three of the vaccinates challenged (13%) had a lymphocyte decrease of over 60% which was 5.2 fold less in the vaccinates; a 2.7 fold less drop in lymphocytes in the vaccinates as observed when considering a lymphocyte drop of 35% or greater and a 2.16 fold less drop in lymphocytes in vaccinates was observed when considering a lymphocyte drop of 25% or greater.

The data indicated that the challenge virus did have an effect on the challenge control dogs and that the vaccination prevented such a severe drop in lymphocytes in the immunized animals.

### 4. Symptoms of Disease

The symptoms of disease noted in this challenge experiment were not as prevalent as in previous experimentation. However, the challenge control dogs did demonstrate more symptoms than did the vaccinate group. A symptom of disease index was devised by dividing the number of symptoms observed per test group by the total number of observations made. For example, 3 symptoms were observed in the intramuscularly vaccinated dogs. A total of 61 observations were made. Therefore, the symptoms index was 3/61 or 0.049. Table 5-1 summarizes the symptom index per group.

**TABLE 5-1**

| Test Group | Observed Symptoms | Number of Observations | Symptom Index |
|---|---|---|---|
| Combined Vacc. | 7 | 131 | 0.053 |
| Controls | 42 | 57 | 0.737 |

A reduction in the symptom index of 92.8% was obtained when comparing the combined vaccinate group with the challenge control dogs. Although the symptoms of disease were not plentiful in the control animals, the vaccine did significantly prevent as many symptoms in the immunized animals.

Each of the ten challenge control animals demonstrated symptoms so the symptom index does not reflect a severe disease syndrome in just a few control dogs. Seven of the twenty-three vaccinates demonstrated one symptom of disease each.

### 5. Intestinal Infection - Evaluation by FA Impression Smear Procedure

The evaluation of the degree of infection of the intestinal tract by challenge virus in the control dogs and vaccinates is the most critical parameter for determining the effectiveness of a Canine Coronavirus vaccine. Intestines were removed and gently washed in cool tap water. The sections were opened with a scalpel and scrapings were taken. Scrapings were placed directly or exfoliated on glass slides from the intestinal epithelium of the test animals. Sampling occurred at appoximately the same location on each animal. The slides were fixed twice in 100% acetone at 25° C for 30 minutes. Specifically labeled Canine Coronavirus conjugate was used to stain each slide for 1 hour at 37° C. The slides were washed in a carbonate buffer wash and observed with a 50W mercury (HBO) light source. The results are presented in Tables 5-2 through 5-6. Each field was observed and a negative to 4+ fluorescent value was given.

**TABLE 5-2**

| Observations of Intestinal Scrapings Impression Slides on Challenge Control Dogs | | | | | | | | |
|---|---|---|---|---|---|---|---|---|
| Days Post Challenge | No. of Samples | No. of Samples per Degree of FA+ | | | | | % of FA+ | Infective Index |
| | | 4+ | 3+ | 2+ | 1+ | 0 | | |
| 5 | 15 | 2 | 0 | 12 | 0 | 1 | 93.3 | 2.13 |
| 5 | 15 | 0 | 0 | 11 | 1 | 4 | 73.3 | 1.47 |
| 5 | 15 | 2 | 0 | 11 | 0 | 2 | 86.7 | 2.0 |
| 5 | 15 | 0 | 0 | 12 | 0 | 3 | 80.0 | 1.6 |
| 6 | 15 | 5 | 0 | 7 | 0 | 3 | 80.0 | 2.26 |
| 6 | 15 | 4 | 0 | 8 | 0 | 3 | 80.0 | 2.13 |
| 6 | 15 | 8 | 0 | 3 | 0 | 4 | 73.3 | 2.53 |
| 7 | 15 | 10 | 0 | 0 | 0 | 5 | 66.7 | 2.67 |
| 7 | 15 | 10 | 0 | 2 | 0 | 4 | 80.0 | 2.93 |
| 7 | 15 | 14 | 0 | 0 | 0 | 1 | 93.3 | 3.73 |
| Total | | 64 | 0 | 66 | 1 | 30 | 889.9 | 23.45 |
| Avg. | | 6.4 | | 6.6 | | 3.0 | 88.99 | 2.345 |

**TABLE 5-3**

| Observation of Intestinal Scraping Impression Slides On Dogs Euthanized 5 days After Challenge | | | | | | | | | |
|---|---|---|---|---|---|---|---|---|---|
| Vaccinate or control | Route of Vaccination | No. of Samples | No. of Samples per Degree of FA+ | | | | | % of FA+ | Infective Index |
| | | | 4+ | 3+ | 2+ | 1+ | 0 | | |
| V | SC | 15 | 0 | 0 | 0 | 2 | 13 | 13 | 0.13 |
| V | SC | 15 | 0 | 0 | 0 | 3 | 12 | 20 | 0.20 |
| V | SC | 15 | 0 | 0 | 0 | 0 | 15 | 0 | 0 |
| V | SC | 15 | 0 | 0 | 0 | 1 | 14 | 6 | 0.06 |
| V | IM | 15 | 0 | 0 | 0 | 2 | 13 | 13 | 0.13 |
| V | IM | 15 | 0 | 0 | 0 | 2 | 13 | 13 | 0.13 |
| V | IM | 15 | 0 | 0 | 0 | 0 | 15 | 0 | 0 |
| C | ― | 15 | 2 | 0 | 12 | 0 | 1 | 93.3 | 2.13 |
| C | ― | 15 | 0 | 0 | 11 | 0 | 4 | 73.3 | 1.47 |
| C | ― | 15 | 2 | 0 | 11 | 0 | 2 | 86.7 | 2.00 |
| C | ― | 15 | 0 | 0 | 12 | 0 | 3 | 80 | 1.6 |

**TABLE 5-4**

| Observation of Intestinal Scraping Impression Slides On Dogs Euthanized 6 Days After Challenge | | | | | | | | | |
|---|---|---|---|---|---|---|---|---|---|
| Vaccinate or control | Route of Vaccination | No. of Samples | No. of Samples per Degree of FA+ | | | | | % of FA+ | Infective Index |
| | | | 4+ | 3+ | 2+ | 1+ | 0 | | |
| V | SC | 15 | 0 | 0 | 0 | 1 | 14 | 6.7 | .067 |
| V | SC | 15 | 0 | 0 | 0 | 0 | 15 | 0 | 0 |
| V | SC | 15 | 0 | 0 | 0 | 5 | 10 | 33.3 | 0.33 |
| V | SC | 15 | 0 | 0 | 0 | 4 | 11 | 26.7 | 0.267 |
| V | IM | 15 | 0 | 0 | 0 | 0 | 15 | 0 | 0 |
| V | IM | 15 | 0 | 0 | 0 | 0 | 15 | 0 | 0 |
| V | IM | 15 | 0 | 0 | 0 | 0 | 15 | 0 | 0 |
| V | IM | 15 | 0 | 0 | 0 | 0 | 15 | 0 | 0 |
| C | ― | 15 | 5 | 0 | 7 | 0 | 3 | 80.0 | 2.27 |
| C | ― | 15 | 4 | 0 | 8 | 0 | 3 | 80.0 | 2.13 |
| C | ― | 15 | 8 | 0 | 3 | 0 | 4 | 73.3 | 2.53 |
| Sentinel Control | ― | 15 | 0 | 0 | 0 | 0 | 15 | 0 | 0 |

**TABLE 5-5**

| Observation of Intestinal Scraping Impression Slides On Dogs Euthanized 7 Days After Challenge | | | | | | | | | |
|---|---|---|---|---|---|---|---|---|---|
| Vaccinate or control | Route of Vaccination | No. of Samples | No. of Samples per Degree of FA+ | | | | | % of FA+ | Infective Index |
| | | | 4+ | 3+ | 2+ | 1+ | 0 | | |
| V | SC | 15 | 0 | 0 | 0 | 1 | 14 | 6.67 | .067 |
| V | IM | 15 | 0 | 0 | 0 | 8 | 7 | 53.3 | .533 |
| V | IM | 15 | 0 | 0 | 0 | 2 | 13 | 13.3 | .133 |
| V | IM | 15 | 0 | 0 | 0 | 3 | 12 | 20.0 | .2 |
| V | IM | 15 | 0 | 0 | 0 | 0 | 15 | 0 | 0 |
| V | IM | 15 | 0 | 0 | 0 | 0 | 15 | 0 | 0 |
| V | IM | 15 | 0 | 0 | 0 | 0 | 2 | 13 | .133 |
| C | ― | 15 | 10 | 0 | 0 | 0 | 5 | 66.7 | 2.67 |
| C | ― | 15 | 10 | 0 | 0 | 2 | 3 | 80 | 2.93 |
| C | ― | 15 | 14 | 0 | 0 | 0 | 1 | 93.3 | 3.73 |
| Sentinel Control | ― | 15 | 0 | 0 | 0 | 0 | 15 | 0 | 0 |

**TABLE 5-6**

| Infective Index Comparison Indicating Reduction in Intestinal Infection | | | |
|---|---|---|---|
| Test Group | Infective Index per Day of Euthanizing | | |
| | 5 | 6 | 7 |
| SC Vaccinates | 0.0975 | 0.132 | 0.067 |
| IM Vaccinates | 0.13 | 0 | 0.217 |
| Combined Groups | 0.108 | 0.066 | 0.187 |
| Challenge Controls | 1.8 | 2.31 | 3.11 |
| Percent Reduction Combined Group vs Controls | 94% | 97% | 94% |

The challenge control infective index ranged from 1.47 to 3.73 with an average of 2.345, whereas the combined vaccinate group of 22 dogs had an infective index of 0.103. These data indicate that a reduction of intestinal infection of 95.6% was achieved in the vaccinates.

### EXAMPLE 6

### This example illustrates Canine Coronavirus as a combination product.

A pilot serial of vaccine was batched with Canine Coronavirus and Canine Parvovirus as components as follows:

| | |
|---|---|
| CCV fluids | 4.5 liters |
| CPV fluids | 2.0 liters |
| Stabilizer | 2.2 liters |
| Fetal Calf Serum | 0.1 liters |
| 1 N NaOH | 0.08 liters |

The materials were blended under refrigeration and bottled aseptically in a 1 ml. dose. The bottles of vaccine were then subjected to desiccation. The virus vields were as follows:

| | |
|---|---|
| CCV | 10^{5.4}FAID₅₀/dose |
| CPV | 10^{6.5}FAID₅₀/dose |

It is quite feasible to routinely produce and batch CCV as a combination product.

This portion of the study was designed to determine if antigen interference would occur when Canine Coronavirus and Canine Parvovirus were combined for administration as a combination vaccine.

The experimental design can be found in Table 6-1. Three groups of dogs were used. Group I was vaccinated with a full field dose of Canine Coronavirus vaccine (1 ml.). Group II was vaccinated with a full field dose of Canine Corona-Parvovirus vaccine (1 ml.). The third group was vaccinated with a full field dose of Canine Parvovirus (1 ml.). Portions of each group were vaccinated intramuscularly (IM) while the remaining dogs were vaccinated subcutaneously (SC). The dogs were bled on day 0 and also 21 days after vaccination. Sentinel controls were also bled on day 0 and day 21.

**TABLE 6-1**

| Antigen Blockage Study Experimental Design | | | |
|---|---|---|---|
| Treatment | Group I | Group II | Group III |
| Prevaccination | 4 vaccinated SC | 3 vaccinated SQ | 3 vaccinated SQ |
| Bleeding and Vaccination (day 0) | 4 vaccinated IM | 5 vaccinated IM | 4 vaccinated IM |
| Post vaccination bleeding (day 21) | bled | bled | bled |

Five replicate titrations were performed on each virus fraction. The data indicated a virus input of 4.5 logs of Coronavirus for Group I, 5.5 logs of Canine Parvovirus for Group II, and 4.4 logs of Canine Coronavirus with 5.5 logs of Canine Parvovirus for Group II.

### 1. Test Group I - Canine Coronavirus Vaccinate

The data indicated that a geometric mean titer of 1:6498 is obtained against Canine Coronavirus.

### 2. Test Group II-Canine Corona-Parvovirus Vaccinate

The dogs in test Group II responded to a geometric mean titer of 1:87,222 against Canine Coronavirus and a geometric mean titer of 1:58,579 against Canine Parvovirus 21 days after receiving 1 field dose of vaccine.

### 3. Test Group III-Canine Parvovirus Vaccinate

A geometric mean titer of 1:65,893 was obtained in this test group against Canine Parvovirus.

The results are summarized in Table 6-2.

**TABLE 6-2**

| Post Serological Canine Corona and Parvo Response Arithmetic and Geometric Means Summary | | | | | |
|---|---|---|---|---|---|
| Group | Vaccine | Post Vaccination Antibody Titer | | | |
| | | AM* | | GM** | |
| | | CCV | CPV | CCV | CPV |
| I | CCV | 64,111 | ― | 6,498 | ― |
| II | CCV-CPV | 163,436 | 128,172 | 87,222 | 58,579 |
| III | CPV | ― | 391,982 | ― | 65,893 |

| | | | | | |
|---|---|---|---|---|---|
| * Arithmetic Mean | | | | | |
| ** Geometric Mean | | | | | |

### Analysis for Antigen Blockage

### a. Canine Coronavirus (See Table 6-2)

No antigen blockage was noted for the Canine Coronavirus. A geometric mean antibody titer of 1:87,222 was achieved with the combination product as compared to a geometric mean antibody level of 1:6498 with the monovalent product against Canine Coronavirus. A potentiation effect is achieved with the Canine Coronavirus in combination. A 13.4 fold increase in Canine Coronavirus antibody titer is realized when comparing the combination product to the monovalent products.

### b. Canine Parvovirus

There was no significant difference in antibody titers against Canine Parvovirus when comparing the combination product with the monovalent product. A geometric mean titer of 1:58,579 with the combination product compares well with a geometric mean titer of 1:65,893 with the monovalent product. No antigen blockage was observed.

### EXAMPLE 7

### This example illustrates canine coronavirus cross neutralization between five various isolates.

Studies were conducted to determine the cross neutralization relationships (serum neutralization of virus) between various isolates of canine coronavirus. Specific antiserum to each individual isolate was prepared in susceptible dogs. Serum neutralization (SN) tests with the various isolates at constant levels or titers were run against twofold dilutions of the specific antisera. In other words the antibody induced in the dogs by a specific isolate was evaluated as to the antibody's ability to neutralize various other virus isolates as well as the identical virus isolate used to induce the antibody. A virus isolate which induced antibody which will neutralize the most virus isolates is the best vaccine virus candidate. Results of those tests are depicted in the following table. The specific antisera are presented vertically against the various isolates listed horizontally. The reciprocal SN level is expressed.

| Antisera produced by indicated CCV | Antibody level against the virus inducing antibody formation | Antibody levels against other viruses | | | | |
|---|---|---|---|---|---|---|
| | | I-171 | K-378 | S-378 | A76-5 | ATCC VR 2068 |
| I-171 | 89 | - | 128 | 89 | 20 | 45 |
| K-378 | 45 | 22 | ― | 89 | 3 | 40 |
| S-378 | 178 | 178 | 148 | ― | 6 | 37 |
| A76-5 | 11 | 45 | 32 | 64 | ― | 50 |
| ATCC No. VR 2068 | 45 | 64 | 128 | 54 | 25 | ― |

The data was analyzed from the standpoint of the antibody which would neutralize all five viruses. The antibody induced by the ATCC No. VR2068 virus isolate neutralized the other four viruses to a significant degree.

The A76-5 antiserum and virus proved to be the least cross reactive of the five isolates tested. The ATCC VR 2068 proved to have a significant level of cross, reactivity with this virus isolate.

The data also indicates that there are no major serological differences among these five canine coronaviruses. The I-171 and ATCC VR 2068 viruses are indicated as the best choices for vaccine strains; however due to the cross neutralization between all the isolates any of the canine coronaviruses could be used as an antigen.

## Claims (Claims for the following Contracting State(s): BE, CH, LI, DE, FR, GB, LU, NL, SE)

1. Attenuated live canine coronavirus which is fully adapted to reproduction and growth in cell culture, which does not produce disease when administered to a dog and which, when administered to a dog by injection, infects intestinal epithelium in the dog to produce localised immunity to canine coronavirus in the intestines.

2. Attenuated live canine coronavirus as claimed in claim 1 which is prepared by passing virulent canine coronavirus in feline cells.

3. Attenuated live canine coronavirus as claimed in claim 1 or 2 which is prepared by passing virulent canine coronavirus in feline cells between 8 and 25 times at a low virus to cell ratio of about 1:1,000 to 1:10,000 as measured by the FAID₅₀ method.

4. Attenuated live canine coronavirus identified as ATCC No. VR-2068.

5. A method of propagating and harvesting attenuated live canine coronavirus comprising the steps of inoculating a primary or established cell line of feline or canine origin in cell suspension or cell monolayers with an amount of attenuated live canine coronavirus sufficient to achieve a minimum multiplicity of infection (MOI) ratio of at least 1:100 wherein the attenuated live canine coronavirus has been attenuated by passing virulent canine coronavirus in feline cells at least 8 times and wherein the cells are present in an amount sufficient to form a confluent monolayer of the cells within about 48 hours or less of inoculation; culturing in a fluid medium a tight or crowded monolayer of the cells for a period of time less than 96 hours after inoculation sufficient to provide infectivity titers of at least 3 logs of particles of virus per ml as measured by the direct FAID₅₀ method; and harvesting the propagated virus from the cellular material, the fluids or both the cellular material and the fluids.

6. A method as claimed in claim 5 wherein the amount of inoculated attenuated live canine coronavirus is sufficient to achieve an MOI ratio of at least 1:75.

7. A method as claimed in claim 5 wherein the amount of inoculated attenuated live canine coronavirus is sufficient to achieve an MOI ratio of at least 1:10.

8. A method as claimed in any one of claims 5 to 7 wherein the inoculated cells are cultured for a period of time less than about 48 hours.

9. A method as claimed in any one of claims 5 to 7 wherein the inoculated cells are cultured for a period of time less than about 24 hours.

10. A method as claimed in any one of claims 5 to 7 wherein the inoculated cells are cultured for a period of time no greater than about 18 hours.

11. A method as claimed in any one of claims 5 to 10 which includes the step of absorbing the virus on the cells for less than about 300 minutes prior to culturing.

12. A vaccine composition for vaccination of dogs comprising an attenuated live canine coronavirus as defined in claims 1-3 obtainable by the method defined in any one of claims 5 to 11 in an amount sufficient to protect the dog from infection by virulent canine coronavirus and a non-toxic pharmaceutically acceptable carrier.

## Claims (Claims for the following Contracting State(s): AT)

1. . A method for obtaining attenuated live canine coronavirus which is fully adapted to reproduction and growth in cell culture, which does not produce disease when administered to a dog and which, when administered to a dog by injection, infects intestinal epithelium in the dog to produce localised immunity to canine coronavirus in the intestines, which comprises passing virulent canine coronavirus in feline cells.

2. A method as claimed in claim 1 which comprises passing virulent canine coronavirus in feline cells between 8 and 25 times at a low virus to cell ratio of about 1:1,000 to 1:10,000 as measured by the FAID₅₀ method.

3. A method of propagating and harvesting attenuated live canine coronavirus comprising the steps of inoculating a primary or established cell line of feline or canine origin in cell suspension or cell monolayers with an amount of attenuated live canine coronavirus sufficient to achieve a minimum multiplicity of infection (MOI) ratio of at least 1:100 wherein the attenuated live canine coronavirus has been attenuated by passing virulent canine coronavirus in feline cells at least 8 times and wherein the cells are present in an amount sufficient to form a confluent monolayer of the cells within about 48 hours or less of inoculation; culturing in a fluid medium a tight or crowded monolayer of the cells for a period of time less than 96 hours after inoculation sufficient to provide infectivity titers of at least 3 logs of particles of virus per ml as measured by the direct FAID₅₀ method; and harvesting the propagated virus from the cellular material, the fluids or both the cellular material and the fluids.

4. A method as claimed in claim 3 wherein the amount of inoculated attenuated live canine coronavirus is sufficient to achieve an MOI ratio of at least 1:75.

5. A method as claimed in claim 3 wherein the amount of inoculated attenuated live canine coronavirus is sufficient to achieve an MOI ratio of at least 1:10.

6. A method as claimed in any one of claims 3 to 5 wherein the inoculated cells are cultured for a period of time less than about 48 hours.

7. A method as claimed in any one of claims 3 to 5 wherein the inoculated cells are cultured for a period of time less than about 24 hours.

8. A method as claimed in any one of claims 3 to 5 wherein the inoculated cells are cultured for a period of time no greater than about 18 hours.

9. A method as claimed in any one of claims 3 to 8 which includes the step of absorbing the virus on the cells for less than about 300 minutes prior to culturing.

## Patentansprüche (Patentansprüche für folgende(n) Vertragsstaat(en): BE, CH, LI, DE, FR, GB, LU, NL, SE)

1. Attenuierter lebender caniner Coronavirus, welcher zur Gänze zur Reproduktion und zum Wachstum in Zellkultur adaptiert ist, welches nicht Krankheit hervorruft, wenn an einen Hund verabreicht und welches, wenn an einen Hund durch Injektion verabreicht, das Darmepithelium des Hundes infiziert, um lokale Immunität im Darm gegenüber caninem Coronavirus zu erzeugen.

2. Attenuierter lebender caniner Coronavirus wie in Anspruch 1 beansprucht, welcher durch Passagieren des virulenten caninen Coronavirus in felinen Zellen hergestellt wird.

3. Attenuierter lebender caniner Coronavirus wie in Anspruch 1 oder 2 beansprucht, welcher durch Passagieren des virulenten caninen Coronavirus in felinen Zellen zwischen 8 und 25 mal bei einem geringen Virus zu Zellenverhältnis von etwa 1 : 1000 zu 1 : 10 000, gemessen nach der FAID₅₀-Methode, hergestellt wird.

4. Attenuierter lebender caniner Coronavirus, identifiziert als ATCC Nr. VR-2068.

5. Verfahren zum Propagieren und zum Ernten von attenuiertem lebenden caninen Coronavirus, umfassend die Schritte der Beimpfung einer ersten oder etablierten Zelllinie felinen oder caninen Ursprungs in Zellsuspension oder Zellmonoschichten mit einer Menge von attenuiertem lebenden caninen Coronavirus ausreichend zum Erzielen einer minimalen Multiplizität des Infektionsverhältnisses (MOI)von zumindest 1 : 100, wobei der attenuierte lebende canine Coronavirus attenuiert wurde durch zumindest 8 maliges Passagieren des virulenten caninen Coronavirus in felinen Zellen zumindest 8 mal, und wobei die Zellen in einer ausreichenden Menge vorhanden sind, um eine konfluente Monoschicht von Zellen innerhalb von etwa 48 Stunden oder weniger nach Inokulation zu bilden; Kultivieren einer dichten oder gehäuften Monoschicht von Zellen in einem flüssigen Medium in einem Zeitraum von weniger als 96 Stunden nach Inokulation, ausreichend, um einen Infektionstiter von zumindest 3 logs von Viruspartikeln pro ml, gemessen mit der direkten FAID₅₀-Methode, zu bilden; und Ernten des propagierten Virus von dem zellulären Material, den Flüssigkeiten oder sowohl von zellulärem Material als auch von den Flüssigkeiten.

6. Verfahren wie in Anspruch 5 beansprucht, wobei die Menge an inokuliertem, attenuiertem lebenden caninen Coronavirus ausreichend ist, um ein MOI-Verhältnis von zumindest 1 : 75 zu erreichen.

7. Verfahren wie in Anspruch 5 beansprucht, wobei die Menge an inokuliertem, attenuiertem lebenden caninen Coronavirus ausreichend ist, um ein MOI-Verhältnis von zumindest 1 : 10 zu erreichen.

8. Verfahren wie in einem der Ansprüche 5 bis 7 beansprucht, wobei die inokulierten Zellen über einen Zeitraum von weniger als etwa 48 Stunden kultiviert werden.

9. Verfahren wie in einem der Ansprüche 5 bis 7 beansprucht, wobei die inokulierten Zellen über einen Zeitraum von weniger als etwa 24 Stunden kultiviert werden.

10. Verfahren wie in einem der Ansprüche 5 bis 7 beansprucht, wobei die inokulierten Zellen über einen Zeitraum von nicht größer als etwa 18 Stunden kultiviert werden.

11. Verfahren wie in einem der Ansprüche 5 bis 10 beansprucht, welches den Schritt der Virusabsorption an den Zellen von weniger als etwa 300 Minuten vor der Kultivierung beinhaltet.

12. Impfstoff-Zusammensetzung zur Impfung von Hunden enthaltend einen attenuierten lebenden caninen Coronavirus wie in den Ansprüchen 1 bis 3 definiert, erhältlich durch das in einem der Ansprüche 5 bis 11 definierte Verfahren, in einer ausreichenden Menge, um den Hund vor Infektion durch virulenten caninen Coronavirus zu schützen, und einen nicht-toxischen pharmazeutisch annehmbaren Träger.

## Patentansprüche (Patentansprüche für folgende(n) Vertragsstaat(en): AT)

1. Verfahren zum Erhalten von attenuiertem lebenden caninen Coronavirus, welches zur Gänze zur Reproduktion und zum Wachstum in zellkultur adaptiert ist, welches nicht Krankheit hervorruft, wenn an einen Hund verabreicht und welches, wenn an einen Hund durch Injektion verabreicht, das Darmepithelium des Hundes infiziert, um lokale Immunität im Darm gegenüber caninem Coronavirus zu erzeugen, welches Passagieren von virulentem caninen Coronavirus in felinen Zellen umfasst.

2. Verfahren wie in Anspruch 1 beansprucht, welches das Passagieren von virulentem caninen Coronavirus in felinen Zellen zwischen 8 und 25 mal bei einem geringen Virus zu Zellenverhältnis von etwa 1 : 1000 zu 1 : 10 000, gemessen nach der FAID₅₀-Methode, umfasst.

3. Verfahren zum Propagieren und zum Ernten von attenuiertem lebenden caninen Coronavirus, umfassend die Schritte der Beimpfung einer ersten oder etablierten Zelllinie felinen oder caninen Ursprungs in Zellsuspension oder Zellmonoschichten mit einer Menge von attenuiertem lebenden caninen Coronavirus ausreichend zum Erzielen einer minimalen Multiplizität des Infektionsverhältnisses (MOI)von zumindest 1 : 100, wobei der attenuierte lebende canine Coronavirus attenuiert wurde durch zumindest 8 maliges Passagieren des virulenten caninen Coronavirus in felinen Zellen zumindest 8 mal, und wobei die Zellen in einer ausreichenden Menge vorhanden sind, um eine konfluente Monoschicht von Zellen innerhalb von etwa 48 Stunden oder weniger nach Inokulation zu bilden; Kultivieren einer dichten oder gehäuften Monoschicht von Zellen in einem flüssigen Medium in einem Zeitraum von weniger als 96 Stunden nach Inokulation, ausreichend, um einen Infektionstiter von zumindest 3 logs von Viruspartikeln pro ml, gemessen mit der direkten FAID₅₀-Methode, zu bilden; und Ernten des propagierten Virus von dem zellulären Material, den Flüssigkeiten oder sowohl von zellulärem Material als auch von den Flüssigkeiten.

4. Verfahren wie in Anspruch 3 beansprucht, wobei die Menge an inokuliertem, attenuiertem lebenden caninen Coronavirus ausreichend ist, um ein MOI-Verhältnis von zumindest 1 : 75 zu erreichen.

5. Verfahren wie in Anspruch 3 beansprucht, wobei die Menge an inokuliertem, attenuiertem lebenden caninen Coronavirus ausreichend ist, um ein MOI-Verhältnis von zumindest 1 : 10 zu erreichen.

6. Verfahren wie in einem der Ansprüche 3 bis 5 beansprucht, wobei die inokulierten Zellen über einen Zeitraum von weniger als etwa 48 Stunden kultiviert werden.

7. Verfahren wie in einem der Ansprüche 3 bis 5 beansprucht, wobei die inokulierten Zellen über einen Zeitraum von weniger als etwa 24 Stunden kultiviert werden.

8. Verfahren wie in einem der Ansprüche 3 bis 5 beansprucht, wobei die inokulierten Zellen über einen Zeitraum von nicht größer als etwa 18 Stunden kultiviert werden.

9. Verfahren wie in einem der Ansprüche 3 bis 8 beansprucht, welches den Schritt der Virusabsorption an den Zellen von weniger als etwa 300 Minuten vor der Kultivierung beinhaltet.

## Revendications (Revendications pour l'(les) Etat(s) contractant(s) suivant(s): BE, CH, LI, DE, FR, GB, LU, NL, SE)

1. Coronavirus canin vivant atténué qui est tout à fait capable de se reproduire et de se développer en culture cellulaire, qui ne provoque pas de maladie lorsqu'il est administré à un chien et qui, lorsqu'il est administré à un chien par injection, infecte l'épithélium intestinal du chien pour produire une immunité localisée contre un coronavirus canin dans les intestins.

2. Coronavirus canin vivant atténué selon la revendication 1 qui est préparé en faisant passer un coronavirus canin virulent dans des cellules de félin.

3. Coronavirus canin vivant atténué selon la revendication 1 ou 2 qui est préparé en faisant passer un coronavirus canin virulent dans des cellules de félin entre 8 et 25 fois à un rapport du virus à la cellule peu élevé d'environ 1:1000 à 1:10 000, tel que mesuré par le procédé FAID₅₀.

4. Coronavirus canin vivant atténué identifié comme étant l'ATCC No. VR-2068.

5. Procédé de propagation et de récolte de coronavirus canin vivant atténué comprenant les étapes d'inoculation d'une lignée cellulaire primaire ou établie d'origine féline ou canine dans une suspension cellulaire ou des couches monocellulaires avec une quantité de coronavirus canin vivant atténué suffisante pour atteindre un taux minimal de multiplicité d'infection (MOI) d'au moins 1:100, **caractérisé en ce que** le coronavirus canin vivant atténué a été atténué en faisant passer un coronavirus canin virulent dans des cellules de félin au moins 8 fois et **en ce que** les cellules sont présentes en une quantité suffisante pour former une monocouche agglomérée de cellules endéans les 48 heures ou moins de l'inoculation; mise en culture dans un milieu liquide d'une monocouche serrée ou dense des cellules pendant un laps de temps inférieur à 96 heures suivant l'inoculation suffisant pour procurer des titres d'infectivité d'au moins 3 logs de particules de virus par ml, comme mesuré par le procédé de FAID₅₀ direct; et récolte du virus propagé à partir du matériau cellulaire ou des fluides ou du matériau cellulaire et des fluides.

6. Procédé selon la revendication 5 **caractérisé en ce que** la quantité de coronavirus canin vivant atténué est suffisante pour atteindre un taux de MOI d'au moins 1:75.

7. Procédé selon la revendication 5 **caractérisé en ce que** la quantité de coronavirus canin vivant atténué est suffisante pour atteindre un taux de MOI d'au moins 1:10.

8. Procédé selon l'une quelconque des revendications 5 à 7 **caractérisé en ce que** les cellules inoculées sont mises en culture pendant un laps de temps inférieur à environ 48 heures.

9. Procédé selon l'une quelconque des revendications 5 à 7 **caractérisé en ce que** les cellules inoculées sont mises en culture pendant un laps de temps inférieur à environ 24 heures.

10. Procédé selon l'une quelconque des revendications 5 à 7 **caractérisé en ce que** les cellules inoculées sont mises en culture pendant un laps de temps ne dépassant pas environ 18 heures.

11. Procédé selon l'une quelconque des revendications 5 à 10 qui comprend l'étape d'absorption du virus sur les cellules pendant moins d'environ 300 minutes avant la mise en culture.

12. Composition de vaccin pour la vaccination de chiens comprenant un coronavirus canin vivant atténué selon les revendications 1 à 3 pouvant être obtenu suivant le procédé selon l'une quelconque des revendication 5 à 11 en une quantité suffisante pour protéger un chien contre une infection par un coronavirus canin virulent et excipient non toxique pharmaceutiquement acceptable.

## Revendications (Revendications pour l'(les) Etat(s) contractant(s) suivant(s): AT)

1. Procédé pour obtenir un coronavirus canin vivant atténué qui est tout à fait capable de se reproduire et de se développer en culture cellulaire, qui ne provoque pas de maladie lorsqu'il est administré à un chien et qui, lorsqu'il est administré à un chien par injection, infecte l'épithélium intestinal du chien pour produire une immunité localisée contre un coronavirus canin dans les intestins, qui comprend le passage d'un coronavirus canin virulent dans des cellules de félin.

2. Procédé selon la revendication 1 qui comprend le passage d'un coronavirus canin virulent dans des cellules de félin entre 8 et 25 fois à un rapport du virus à la cellule peu élevé d'environ 1:1000 à 1:10 000, tel que mesuré par le procédé FAID₅₀.

3. Procédé de propagation et de récolte de coronavirus canin vivant atténué comprenant les étapes d'inoculation d'une lignée cellulaire primaire ou établie d'origine féline ou canine dans une suspension cellulaire ou des couches monocellulaires avec une quantité de coronavirus canin vivant atténué suffisante pour atteindre un taux minimal de multiplicité d'infection (MOI) d'au moins 1:100, **caractérisé en ce que** le coronavirus canin vivant atténué a été atténué en faisant passer un coronavirus canin virulent dans des cellules de félin au moins 8 fois et **en ce que** les cellules sont présentes en une quantité suffisante pour former une monocouche agglomérée de cellules endéans les 48 heures ou moins de l'inoculation; mise en culture dans un milieu liquide d'une monocouche serrée ou dense des cellules pendant un laps de temps inférieur à 96 heures suivant l'inoculation suffisant pour procurer des titres d'infectivité d'au moins 3 logs de particules de virus par ml, comme mesuré par le procédé de FAID₅₀ direct; et récolte du virus propagé à partir du matériau cellulaire ou des fluides ou du matériau cellulaire et des fluides.

4. Procédé selon la revendication 3 **caractérisé en ce que** la quantité de coronavirus canin vivant atténué est suffisante pour atteindre un taux de MOI d'au moins 1:75.

5. Procédé selon la revendication 3 **caractérisé en ce que** la quantité de coronavirus canin vivant atténué est suffisante pour atteindre un taux de MOI d'au moins 1:10.

6. Procédé selon l'une quelconque des revendications 3 à 5 **caractérisé en ce que** les cellules inoculées sont mises en culture pendant un laps de temps inférieur à environ 48 heures.

7. Procédé selon l'une quelconque des revendications 3 à 5 **caractérisé en ce que** les cellules inoculées sont mises en culture pendant un laps de temps inférieur à environ 24 heures.

8. Procédé selon l'une quelconque des revendications 3 à 5 **caractérisé en ce que** les cellules inoculées sont mises en culture pendant un laps de temps ne dépassant pas environ 18 heures.

9. Procédé selon l'une quelconque des revendications 3 à 8 qui comprend l'étape d'absorption du virus sur les cellules pendant moins d'environ 300 minutes avant la mise en culture.
